# FASCICULE DE BREVET EUROPEEN

(11) **EP 0 708 754 B1**
(45) Date de publication et mention de la délivrance du brevet: **22.10.1997**
(21) Numéro de dépôt: 94922918.1
(22) Date de dépôt: 13.07.1994
(51) Int. Cl.: C07C 269/06, C07C 249/02, C07C 317/28

(54) **PROCEDE DE PREPARATION DE N-CARBONYLARYLIMINES UTILES DANS LA SYNTHESE DE TAXOIDES THERAPEUTIQUEMENT ACTIFS**
VERFAHREN ZUR HERSTELLUNG VON N-CARBONYLARYLIMINE ALS ZWISCHENPRODUKTE IN DER SYNTHESE VON THERAPEUTISCH AKTIVEN TAXOL-DERIVATEN
PROCESS FOR THE PREPARATION OF N-CARBONYLARYLIMINES USEFUL FOR THE SYNTHESIS OF THERAPEUTICALLY ACTIVE TAXOIDS

(30) Priorité: 16.07.1993 FR 9308752
(43) Date de publication de la demande: 01.05.1996
(73) Titulaire: RHONE-POULENC RORER S.A., 92160 Antony (FR)
(72) Inventeur: BERNARD, Didier, F-69009 Lyon (FR); LEON, Patrick, F-69160 Tassin-La-Demi-Lune (FR)
(74) Mandataire: Pilard, Jacques
(86) Numéro de dépôt international: FR9400880
(87) Numéro de publication internationale: WO9502576

(56) Documents cités:
- ARCHIV DER PHARMAZIE. vol. 307, no. 8 , Aoüt 1974 , WEINHEIM DE pages 653 - 655 P. MESSINGER ET AL 'NOTIZ ZUR SYNTHESE VON ALPHA-AMINO UND ALPHA-AMIDOSULFONEN'

## Description

La présente invention concerne un procédé de préparation de N-carbonylarylimines de formule générale : 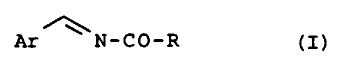 dans laquelle Ar représente un radical aryle et R représente un radical phényle ou t.butoxy.

Dans la formule générale (I), Ar représente un radical aryle et R représente un radical phényle ou t.butoxy.

Plus particulièrement, Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

Plus particulièrement, Ar représente un radical phényle, thiényle-2 ou -3 ou furyle-2 ou -3 éventuellement substitué par un ou plusieurs atomes ou radicaux, identiques ou différents, choisis parmi les atomes d'halogène et les radicaux alcoyles, alcoxy, amino, alcoylamino, dialcoylamino, acylamino, alcoxycarbonylamino et trifluorométhyle.

Plus particulièrement encore, Ar représente un radical phényle éventuellement substitué par un atome de chlore ou de fluor, ou par un radical alcoyle (méthyle), alcoxy (méthoxy), dialcoylamino (diméthylamino), acylamino (acétylamino) ou alcoxycarbonylamino (tert-butoxycarbonylamino) ou thiényle-2 ou -3 ou furyle-2 ou -3.

Selon l'invention, les N-carbonylimines de formule générale (I) peuvent être préparées par action d'une base sur une sulfone de formule générale : 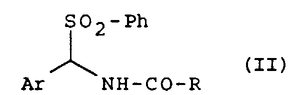 dans laquelle Ar et R sont définis comme précédemment et Ph représente un radical phényle éventuellement substitué, par exemple par un radical méthyle.

Généralement, on opère en présence d'un carbonate de métal alcalin tel que le carbonate de sodium ou de potassium dans un solvant organique choisi parmi les éthers (tétrahydrofuranne) et les hydrocarbures aromatiques (benzène, toluène) à une température comprise entre 50 et 100°C.

La sulfone de formule générale (II) peut être préparée, éventuellement in situ, par action d'un phénylsulfinate alcalin tel que le phénylsulfinate de sodium ou de potassium sur un mélange d'un aldéhyde de formule générale :

Ar-CHO (III)

dans laquelle Ar est défini comme précédemment, et d'un produit de formule générale

H₂N-CO-R (IV)

dans laquelle R est défini comme précédemment en opérant dans un milieu hydro-organique tel qu'un mélange d'eau et d'un alcool aliphatique contenant 1 à 3 atomes de carbone (méthanol, éthanol) en présence d'un acide tel que l'acide formique.

Les N-carbonylimines de formule générale (I) sont particulièrement utiles pour préparer stéréosélectivement les dérivés de la β-phénylisosérine de formule générale : 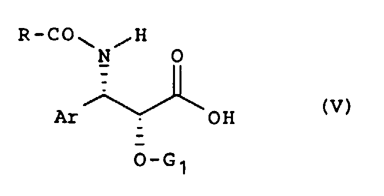 dans laquelle Ar et R sont définis comme précédemment et G₁ représente un groupement protecteur de la fonction hydroxy choisi parmi les radicaux méthoxyméthyle, éthoxy-1 éthyle, benzyloxyméthyle, trichloro-2,2,2 éthoxyméthyle, tétrahydrofurannyle, tétrahydropyrannyle, β-triméthylsilyléthoxyméthyle, trialcoylsilyle dont chaque partie alcoyle contient 1 à 4 atomes de carbone ou un radical -CH₂-Ph₁ dans lequel Ph₁ représente un radical phényle éventuellement substitué par un ou plusieurs atomes ou radicaux, identiques ou différents, choisis parmi les atomes d'halogène et les radicaux alcoyles ou alcoxy contenant 1 à 4 atomes de carbone.

Les dérivés de la β-phénylisosérine de formule générale (V) sont particulièrement utiles pour préparer les taxoïdes thérapeutiquement actifs de formule générale: 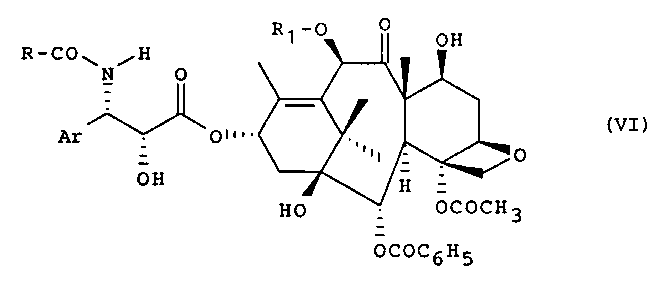 dans laquelle Ar et R sont définis comme précédemment et R₁ représente un atome d'hydrogène ou un radical acétyle dans un procédé qui consiste à faire réagir un produit de formule générale (V) sur un dérivé de la baccatine III ou de la désacétyl-10 baccatine III de formule générale : 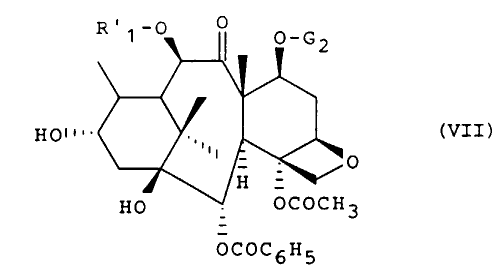 dans laquelle R'₁ représente un radical acétyle ou un groupement protecteur de la fonction hydroxy tel qu'un radical trichloro-2,2,2 éthoxycarbonyle et G₂ représente un groupement protecteur de le fonction hydroxy tel qu'un radical trichloro-2,2,2 éthoxycarbonyle ou trialcoyle silyle, pour obtenir un produit de formule générale : 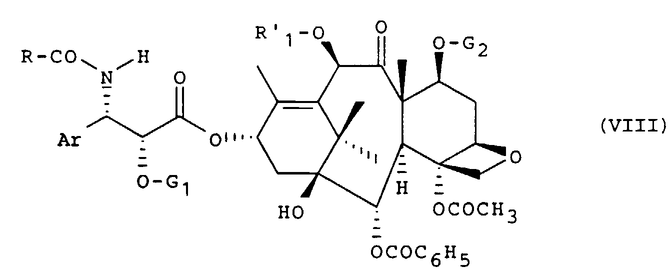 dans laquelle Ar, R, R'₁, G₁ et G₂ sont définis comme précédemment, dont les groupements protecteurs G₁, G₂ et éventuellement R'₁ sont remplacés par des atomes d'hydrogène simultanément ou successivement.

Généralement, l'estérification d'un produit de formule générale (VII) par un produit de formule générale (V) est effectuée en présence d'un agent de condensation tel qu'un carbodiimide comme le dicyclohexylcarbodiimide ou un carbonate réactif comme le pyridyl-2 carbonate et d'un agent d'activation tel qu'une aminopyridine comme la diméthylamino-4 pyridine ou la pyrrolidino-4 pyridine en opérant dans un solvant organique tel qu'un hydrocarbure aromatique (benzène, toluène, xylène, éthylbenzène, isopropylbenzène, chlorobenzène), un éther (tétrahydrofuranne), un nitrile (acétonitrile) ou un ester (acétate d'éthyle) à une température comprise entre 0 et 90°C.

Lorsque G₁ représente un radical méthoxyméthyle, éthoxy-1 éthyle, benzyloxyméthyle, trichloro-2,2,2 éthoxyméthyle, tétrahydrofurannyle, tétrahydropyrannyle, β-triméthylsilyléthoxyméthyle ou trialcoylsilyle dont les radicaux alcoyles contiennent 1 à 4 atomes de carbone, le remplacement des groupements protecteurs G₁, G₂ et éventuellement R'₁ du produit de formule générale (VIII) est effectué soit par le zinc, éventuellement associé au cuivre, en présence d'un acide minéral ou organique tel que l'acide chlorhydrique ou l'acide acétique éventuellement en solution dans un alcool aliphatique contenant 1 à 3 atomes de carbone éventuellement suivi d'un traitement par un acide minéral ou organique tel que l'acide chlorhydrique ou l'acide acétique éventuellement en solution dans un alcool aliphatique contenant 1 à 3 atomes de carbone, lorsque l'un des groupements protecteurs représente un radical trichloro-2,2,2 éthoxycarbonyle soit par traitement par un acide minéral ou organique tel que l'acide chlorhydrique ou l'acide acétique éventuellement en solution dans un alcool aliphatique contenant 1 à 3 atomes de carbone lorsque l'un des groupements protecteurs représente un radical silylé.

Lorsque G₁ représente un radical -CH₂-Ph₁ ou éventuellement benzyloxyméthyle, on effectue d'abord le remplacement des groupements protecteurs G₂ et éventuellement R'₁ par des atomes d'hydrogène dans les conditions décrites ci-dessus pour obtenir le produit de formule générale : 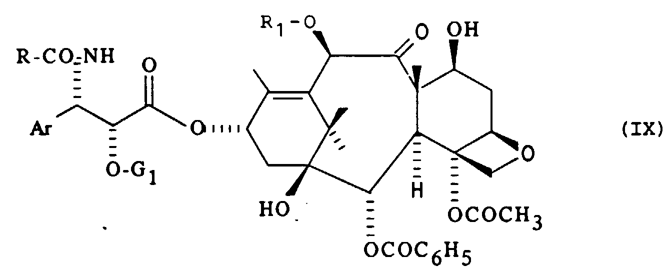 dans laquelle R, Ar et R₁ sont définis comme précédemment dont le groupement Ph₁-CH₂- ou éventuellement benzyloxyméthyle est remplacé par un atome d'hydrogène pour obtenir le produit de formule générale (VI).

Le remplacement du groupement Ph₁-CH₂- ou éventuellement benzyloxyméthyle du produit de formule générale (IX) par un atome d'hydrogène s'effectue généralement par hydrogénolyse au moyen d'hydrogène en présence d'un catalyseur tel que le palladium sur noir en opérant dans un solvant organique tel que l'acide acétique à une température comprise entre 0 et 60°C, de préférence voisine de 40°C. Il peut être avantageux d'opérer sous pression et éventuellement en présence d'une quatité catalytique d'un acide tel que l'acide perchlorique. Le même remplacement s'effectue aussi par l'action de la dichlorodicyanobenzoquinone (DDQ) dans un solvant organique tel que le dichlorométhane ou l'acétonitrile.

Les dérivés du taxane de formule générale (VI) ainsi obtenus peuvent être éventuellement purifiés par application des techniques habituelles.

Les produits de formule générale (V) peuvent être obtenus par action d'une N-carbonylarylimine de formule générale (I) sur l'anion d'un amide optiquement actif d'un acide hydroxyacétique protégé de formule générale : 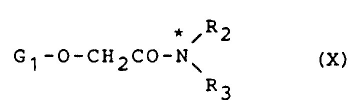 dans laquelle G₁ est défini comme précédemment et 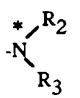 représente le reste d'une base organique optiquement active, suivie de l'hydrolyse du produit ainsi obtenu de formule générale : 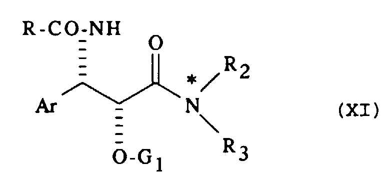 dans laquelle R, Ar, G₁ et 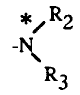 sont définis comme précédemment.

Il est particulièrement avantageux d'utiliser un amide de formule générale (X) dans laquelle 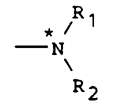 représente un reste L(+)-2,10-camphorsultame de formule :

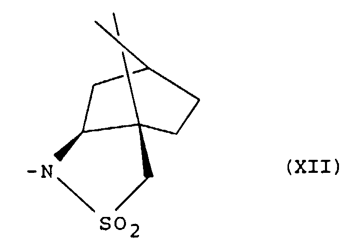

Le procédé est généralement mis en oeuvre en faisant réagir la N-carbonylarylimine de formule générale (I), éventuellement préparée in situ, sur l'amide de l'acide hydroxyacétique protégé préalablement anionisé. L'anionisation s'effectue généralement au moyen d'un amidure de métal alcalin. Parmi les amidures appropriés peuvent être cités le bis(triméthylsilyl) amidure de sodium (NHMDS), de lithium (LHMDS) ou de potassium (KHMDS), le diisopropylamidure de lithium (LDA), le diéthylamidure de lithium (LDEA), le dicyclohexylamidure de lithium (LDCHA), (CH₃)₃SiN(R')Li (R' = alcoyle, cycloalcoyle, aryle) et tBuLi. D'un intérêt tout particulier est le bis(triméthylsilyl) amidure de lithium qui permet d'obtenir un rendement élevé et une excellente stéréosélectivité.

Généralement l'anionisation est effectuée dans un solvant organique inerte tel qu'un éther comme le tétrahydrofuranne à une température inférieure à 0°C et de préférence voisine de -78°C.

L'action du produit de formule générale (I) sur le produit de formule générale (X) préalablement anionisé s'effectue généralement dans le même solvant et à la même température.

Le produit de formule générale (XI) est hydrolysé en produit de formule générale (V) au moyen d'une base minérale telle que la soude, la potasse ou la lithine en milieu aqueux ou hydro-organique. Il est particulièrement avantageux d'opérer dans un mélange tétrahydrofuranne-eau en présence d'eau oxygénée. La température de la réaction est généralement comprise entre -10 et 20°C et de préférence au voisinage de 0°C.

Les exemples suivants illustrent la présente invention.

### EXEMPLE 1

Dans un ballon tricol de 3 litres, muni d'un système d'agitation mécanique à pales, d'un thermomètre et d'une ampoule de coulée, on introduit 1200 cm3 d'eau, 600 cm3 de méthanol, 70,2 g de carbamate de t.butyle (0,6 mole) et 236,1 g de phénylsulfinate de sodium (1,44 mole) puis on ajoute 127,2 g de benzaldéhyde (1,2 mole). On agite à une température voisine de 20°C jusqu'à dissolution totale puis on ajoute, en 15 minutes, 45,2 cm3 d'acide formique pur (d = 1,22) (1,2 mole). Après 30 minutes le milieu se trouble. On poursuit l'agitation pendant 18 heures puis sépare le précipité formé par filtration sur verre fritté. Le précipité est lavé par 200 cm3 d'éther isopropylique puis est séché sous pression réduite. On obtient ainsi, avec un rendement de 69,4%, 144,5 g de N-t.butoxycarbonyl α-phénylsulfonylbenzylamine dont la structure est confirmée par le spectre de résonance magnétique nucléaire du proton et le spectre de masse.

La sulfone ainsi obtenue (6,94 g ; 0,02 mole) est mise en solution dans 60 cm3 de toluène anhydre puis on ajoute 3,31 g de carbonate de potassium (0,024 mole). On chauffe, tout en agitant, à une température voisine de 80°C pendant 3 heures. On ajoute 20 cm3 de toluène. Après refroidissement et filtration du mélange réactionnel, le filtrat est concentré à sec sous pression réduite. On obtient ainsi, avec un rendement de 93%, 3,80 g de N-t.butoxycarbonyl benzylimine sous forme d'une huile incolore dont les caractéristiques sont les suivantes :
- spectre infra-rouge (film) : bandes d'absorption caractéristiques à 3050, 2970, 2925, 1730, 1650, 1590, 1485, 1460, 1320, 1275, 1260, 1155, 1000, 980, 885, 850, 755 et 690 cm⁻¹.
- spectre de RMN du proton (360 MHz ; CDCl₃) : 1,52 (s, 9H) ; 7,39-7,49 (m, 3H) ; 7,84 (m, 2H); 8,80 (s, 1H).

### EXEMPLE 2

Dans un réacteur de 500 cm3, on introduit 10 g de la sulfone obtenue précédemment (28,8 mmoles), 150 cm3 de tétrahydrofuranne anhydre et 18,3 g de carbonate de sodium préalablement séché (172,7 mmoles). On chauffe au reflux pendant 15 heures 20 minutes. Après refroidissement à une température voisine de 5°C et filtration, le filtrat est concentré à sec. On obtient, avec un rendement de 96% après purification, 5,66 g de N-t.butoxycarbonyl benzylimine pure dont les caractéristiques sont identiques à celles du produit obtenu à l'exemple 1.

## Revendications

1. Procédé de préparation d'une N-carbonylarylimine de formule générale : 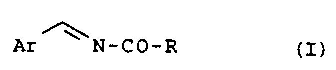 dans laquelle Ar représente un radical aryle et R représente un radical phényle ou t.butoxy caractérisé en ce que l'on désulfone une sulfone de formule générale : 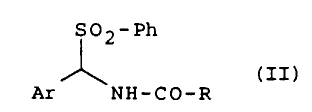 dans laquelle Ar et R sont définis comme précédemment et Ph représente un radical phényle éventuellement substitué.

2. Procédé selon la revendicartion 1 caractérisé en ce que la désulfonation est effectuée en présence d'une base.

3. Procédé selon la revendication 2 caractérisé en ce que la base est choisie parmi les carbonates de métaux alcalins.

4. Procédé selon l'une des revendications 1 à 3 caractérisé en ce que l'on opère dans un solvant organique choisi parmi les éthers et les hydrocarbures aromatiques.

5. Procédé selon l'une des revendications 1 à 4 caractérisé en ce que l'on opère à une température comprise entre 50 et 100°C.

6. Procédé selon l'une des revendications 1 à 5 pour la préparation d'un N-carbonylarylimine de formule générale : 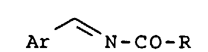 dans laquelle R étant défini comme dans la revendication 1, Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonyl-amino, amino, alcoylamino, dialcoylamino, carboxy, alcoxycarbonyle, carbamoyle, dialcoylcarbamoyle, cyano, nitro et trifluorométhyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles, ou bien Ar représente un radical hétérocyclique aromatique ayant 5 chaînons et contenant un ou plusieurs atomes, identiques ou différents, choisis parmi les atomes d'azote, d'oxygène ou de soufre, éventuellement substitué par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

7. Une sulfone de formule générale : 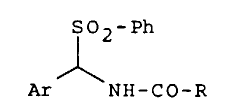 dans laquelle Ar est défini comme dans l'une des revendications 1 ou 6, R représente un radical t.butoxy et Ph représente un radical phényle éventuellement substitué.

## Patentansprüche

1. Verfahren zur Herstellung eines N-Carbonylarylimins der allgemeinen Formel (I) 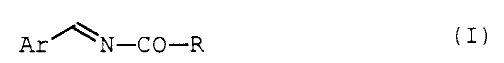 in der Ar einen Rest Aryl darstellt und R einen Rest Phenyl oder tert.-Butoxy bedeutet, dadurch gekennzeichnet, daß man ein Sulfon der allgemeinen Formel 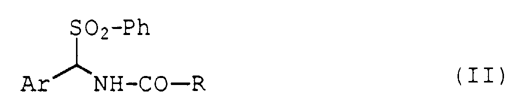 in der Ar und R wie vorstehend definiert sind und Ph einen gegebenenfalls substituierten Rest Phenyl darstellt, desulfoniert.

2. Verfahren nach Anspruch 1, dadurch gekennzeichnet, daß die Desulfonierung in Anwesenheit einer Base durchgeführt wird.

3. Verfahren nach Anspruch 2, dadurch gekennzeichnet, daß die Ba se unter den Carbonaten der Alkalimetalle ausgewählt wird.

4. Verfahren nach einem der Ansprüche 1 bis 3, dadurch gekennzeichnet, daß man in einem organischen Lösungsmittel arbeitet, ausgewählt unter den Ethern und den aromatischen Kohlenwasserstoffen.

5. Verfahren nach einem der Ansprüche 1 bis 4, dadurch gekennzeichnet, daß man bei einer Temperatur zwischen 50 °C und 100 °C arbeitet.

6. Verfahren nach einem der Ansprüche 1 bis 5 zur Herstellung ei nes N-Carbonylarylimins der allgemeinen Formel 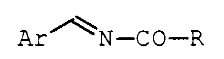 in der R wie in Anspruch 1 definiert ist, Ar einen Rest Phenyl oder α- oder β-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Aroylamino, Alkoxycarbonylamino, Amino, Alkylamino, Dialkylamino, Carboxy, Alkoxycarbonyl, Carbamoyl, Dialkylcarbamoyl, Cyano, Nitro und Trifluormethyl, mit der Maßgabe, daß die Alkylreste und Alkylteile der anderen Reste 1 bis 4 Kohlenstoffatome enthalten, daß die Reste Alkenyl und Alkinyl 2 bis 8 Kohlenstoffatome enthalten und daß die Reste Aryl Reste Phenyl oder α- oder β-Naphthyl sind, oder auch Ar einen aromatischen heterocyclischen Rest mit 5 Ringgliedern darstellt, der ein oder mehrere gleiche oder verschiedene Atome enthalten kann, ausgewählt unter den Atomen von Stickstoff, Sauerstoff oder Schwefel, gegebenenfalls substituiert durch einen oder mehrere gleiche oder verschiedene Substituenten, ausgewählt unter den Halogenatomen (Fluor, Chlor, Brom, Iod) und den Resten Alkyl mit 1 bis 4 Kohlenstoffatomen, Aryl mit 6 bis 10 Kohlenstoffatomen, Alkoxy mit 1 bis 4 Kohlenstoffatomen, Aryloxy mit 6 bis 10 Kohlenstoffatomen, Amino, Alkylamino mit 1 bis 4 Kohlenstoffatomen, Dialkylamino, bei dem jeder Alkylteil 1 bis 4 Kohlenstoffatome enthält, Acylamino, dessen Acylteil 1 bis 4 Kohlenstoffatome enthält, Alkoxycarbonylamino mit 1 bis 4 Kohlenstoffatomen, Acyl mit 1 bis 4 Kohlenstoffatomen, Arylcarbonyl, dessen Arylteil 6 bis 10 Kohlenstoffatome enthält, Cyano, Carboxy, Carbamoyl, Alkylcarbamoyl, dessen Alkylteil 1 bis 4 Kohlenstoffatome enthält, Dialkylcarbamoyl, bei dem jeder Alkylteil 1 bis 4 Kohlenstoffatome enthält, oder Alkoxycarbonyl, dessen Alkoxyteil 1 bis 4 Kohlenstoffatome enthält.

7. Ein Sulfon der allgemeinen Formel 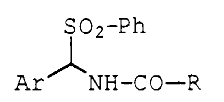 in der Ar wie in einem der Ansprüche 1 oder 6 definiert ist, R einen Rest tert.-Butoxy darstellt und Ph einen gegebenenfalls substituierten Rest Phenyl bedeutet.

## Claims

1. Process for the preparation of an N-carbonylarylimine of general formula: 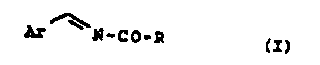 in which Ar represents an aryl radical and R represents a phenyl or t-butoxy radical, characterized in that a sulphone of general formula: 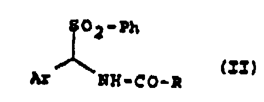 in which Ar and R are defined as above and Ph represents an optionally substituted phenyl radical, is desulphonated.

2. Process according to claim 1, characterized in that the desulphonation is carried out in the presence of a base.

3. Process according to claim 2, characterized in that the base is chosen from alkali metal carbonates.

4. Process according to one of claims 1 to 3, characterized in that it is performed in an organic solvent chosen from ethers and aromatic hydrocarbons.

5. Process according to one of claims 1 to 4, characterized in that it is performed at a temperature between 50 and 100°C.

6. Process according to one of claims 1 to 5 for the preparation of an N-carbonylarylimine of general formula: 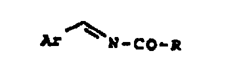 in which, R being defined as in claim 1, Ar represents a phenyl or α- or β-naphthyl radical optionally substituted with one or more atoms or radicals chosen from halogen atoms, (fluorine, chlorine, bromine or iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, it being understood that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals, or alternatively Ar represents a 5-membered aromatic heterocyclic radical containing one or more atoms, which may be identical or different, chosen from nitrogen, oxygen or sulphur atoms, which are optionally substituted with one or more substituents, which may be identical or different, chosen from halogen atoms (fluorine, chlorine, bromine or iodine) and alkyl radicals containing 1 to 4 carbon atoms, aryl radicals containing 6 to 10 carbon atoms, alkoxy radicals containing 1 to 4 carbon atoms, aryloxy radicals containing 6 to 10 carbon atoms, amino radicals, alkylamino radicals containing 1 to 4 carbon atoms, dialkylamino radicals in which each alkyl part contains 1 to 4 carbon atoms, acylamino radicals in which the acyl part contains 1 to 4 carbon atoms, alkoxycarbonylamino radicals containing 1 to 4 carbon atoms, acyl radicals containing 1 to 4 carbon atoms, arylcarbonyl radicals in which the aryl part contains 6 to 10 carbon atoms, cyano, carboxyl and carbamoyl radicals, alkylcarbamoyl radicals in which the alkyl part contains 1 to 4 carbon atoms, dialkylcarbamoyl radicals in which each alkyl part contains 1 to 4 carbon atoms, and alkoxycarbonyl radicals in which the alkoxy part contains 1 to 4 carbon atoms.

7. A sulphone of general formula: 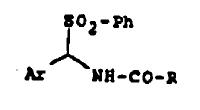 in which Ar is defined as in either of claims 1 and 6, R represents a t-butoxy radical and Ph represents an optionally substituted phenyl radical.
